# EUROPEAN PATENT APPLICATION

(11) **EP 4 289 942 A1**
(43) Date of publication of application: **13.12.2023**
(21) Application number: 22749959.7
(22) Date of filing: 27.01.2022
(51) Int. Cl.: C12N 5/079, A61K 35/30, A61P 27/02, C12M 1/00

(54) **METHOD FOR INDUCING AND PREPARING RETINAL OUTER LAYER CELLS FROM STEM CELLS, AND COMPOSITION FOR PREVENTING OR TREATING RETINAL DISEASES, CONTAINING CELLS PREPARED THEREBY**

(30) Priority: 02.02.2021 KR 20210015038
(71) Applicant: S-BIOMEDICS, Seoul 04797 (KR)
(72) Inventor: CHO, Myung Soo, Hanam-si Gyeonggi-do 12924 (KR); NAM, Seung Taek, Seoul 02789 (KR); EOM, Jang Hyeon, Namyangju-si Gyeonggi-do 12248 (KR)
(74) Representative: J A Kemp LLP
(86) International application number: PCT/KR2022/001533
(87) International publication number: WO 2022/169209

(57) **Abstract**

The present invention relates to a method for differentiating a spherical neural mass (SNM) into retinal outer layer cells, and a composition containing the retinal outer layer cells prepared by the differentiation method. If the differentiation method of the present invention is used, the SNM can be efficiently differentiated into retinal outer layer cells, and thus the present invention can be effectively used in research development and commercialization related thereto.

## Description

### Technical Field

The present invention relates to a method for differentiation and generation of retinal outer layer cells from a spherical neural mass (SNM).

This application claims priority to Korean Patent Application No. 10-2021-0015038 filed in the Korean Intellectual Property Office on 2 February 2021, the disclosure of which is incorporated herein by reference.

### Background Art

The medical definition of blindness is the lack of sensation of light, and such a disease currently affects tens of millions of patients, which is about 0.2-0.5% of the total population worldwide, and causes large personal, social, and economic losses. One of the largest causes of blindness worldwide is the degeneration of retinal photoreceptor cells and retinal pigment epithelial cells, which occurs due to congenital factors or various other causes. This disease includes many diseases, such as retinal hypoplasia, retinal degeneration, aged macular degeneration, diabetic retinopathy, retinitis pigmentosa, congenital retinal atrophy, leber congenital amaurosis, retinal detachment, glaucoma, optic neuropathy, and trauma. There is no drug therapy for the fundamental treatment of these diseases so far, and the regeneration of dysfunctional visual cells and retinal pigment epithelial cells, which are the causes and results of these diseases, into new functional cells and the implantation thereof is considered the most promising treatment. The transplantation of visual cells and retinal pigment epithelial cells is evaluated to prevent blindness or regenerate damaged vision by delaying or suppressing retinal degeneration, regenerating degenerated or degraded retinas and regenerating degenerated retina to enhance retinal functions.

The possibility of treating eye diseases using bone marrow stem cells (BMSs), retinal stem cells (RSCs), embryonic stem cells (ESCs), induced pluripotent stem cells (iPSCs), somatic cell nuclear transfer cells (SCNTs), and the like is emerging. However, research results on the differentiation of stem cells into retinal cells and the cell therapy using the same are insufficient. If these stem cells can be differentiated into retinal cells, 1) the infinite supply of cells for effective cell therapy is possible; 2) a hitherto unknown mechanism of differentiation from embryonic cells and retinal progenitors to retinal cells is identified; 3) retinal differentiation-related genes and molecules are discovered and lesions thereof are identified; 4) a mechanism of development of retinal degenerative disease is identified; and 5) the development of drugs for preventing retinal regeneration and protecting retinal nerves is possible.

Since the establishment of the first human embryonic stem cell lines, human embryonic stem cells have the potential to differentiate into a wide variety of cells, and the differentiated cells are applicable to human cell therapy whereby the diseases occurring in various organs can be treated. In other words, the properties of human embryonic stem cells are accurately identified and human embryonic stem cells are considered as strong candidates that can supply abundant cells to replace dysfunctional cells with new cells during clinical treatment. Differentiated cells constituting various organs derived from human embryonic stem cells have been assumed to have the same properties and functions as corresponding cells formed through a normal differentiation process. Based on this possibility, a differentiation induction method providing an environment similar to the developmental stage has been attempted in pancreatic hormone-expressing endocrine cells, nerve cells, muscle cells, and vascular endothelial cell differentiation. As for even retinal diseases, many attempts have been made to produce photoreceptor cells and retinal pigment epithelial cells, which are representative cells of retinal cells and differentiated from human embryonic stem cells, but the results have been very poor.

A research result reported as one of the largest successes so far is that retinal progenitor cells were effectively induced from human embryonic stem cells, but the differentiation into visual cells from the induced retinal progenitor cells failed (a differentiation rate of less than 0.01%) (Lamba, Proc. Natl. Acad. Sci. USA, 2006; 103: 12769-74).

Accordingly, the development of clinically applicable differentiation methods of visual cells and retinal pigment endothelial cells is needed.

### Disclosure of Invention

### Technical Problem

The present inventors have made intensive endeavors to develop a method for differentiating clinically applicable retinal outer layer cells, that is, photoreceptor cells and retinal pigment epithelial cells. As a result, the present inventors established that the culturing of spherical neural mass single cells and a cystic structure of spherical neural mass together in a single medium result in high efficiency of differentiation into photoreceptor cells and retinal pigment epithelial cells, and thus completed the present invention.

Accordingly, an aspect of the present invention is to provide a method for differentiating a spherical neural mass (SNM) into retinal outer layer cells, the method including:
culturing spherical neural mass single cells and a cystic structure of spherical neural mass together in a single medium; and
differentiating the spherical neural mass into retinal outer layer cells.

Another aspect of the present invention is to provide a pharmaceutical composition for prevention or treatment of a retinal disease, the pharmaceutical composition containing the retinal outer layer cells generated by the differentiation method.

Still another aspect of the present invention is to provide a method for treating a retinal disease, the method including administering, to a subject, the retinal outer layer cells generated by the differentiation method.

Still another aspect of the present invention is to provide use of the retinal outer layer cells, generated by the differentiation method, for treating a retinal disease.

### Solution to Problem

In accordance with an aspect of the present invention, there is provided a method for differentiating a spherical neural mass (SNM) into retinal outer layer cells, the method including:
culturing spherical neural mass single cells and a cystic structure of spherical neural mass together in a single medium; and
differentiating the spherical neural mass into retinal outer layer cells.

As used herein, the term "retinal outer layer cells" refers to photoreceptor cells and retinal pigment epithelium cells, which are present in the outer layer among the cells constituting the retina composed of multiple layers.

As used herein, the term "photoreceptor cells" refers to rod cells and cone cells present in the outer layer of the retina. The photoreceptor cells may be referred to as light-receiving cells, light receptor cells, or the like.

As used herein, the term "retinal pigment epithelial cells" refers to cells constituting the retinal pigment epithelium located in the outermost layer of the retina and in contact with the choroid.

As used herein, the term "spherical neural mass" refers to a spherical form of an aggregate of neural progenitor cells made from pluripotent stem cells, such as human embryonic stem cells or induced pluripotent stem cells, or neural progenitor cells isolated from nervous tissue.

The spherical neural mass used in the present invention may have a size of 300 to 700 um. According to an embodiment of the present invention, the spherical neural mass may have a size of 450 to 550 µm.

The spherical neural mass single cells may be prepared through a step of dissociating the spherical neural mass into single cells. The dissociation into single cells may include fragmentation of the spherical neural mass by using a fine fragmenting tool. The fragmentation using cell separating enzymes may cause damage to many cells, resulting in a low rate of recovery, and thus a physical method may be used. A tool for fragmentation may be any tool that can dissect aggregates with a diameter of 500 um or smaller under a dissecting microscope, and specifically, a finely drawn glass Pasteur pipette, a metal wire with a diameter of 100 um or less, and a metal mesh with micrometer (µm)-sized holes may be used. According to an embodiment of the present invention, the fragmentation may be performed using a tungsten wire.

The fragmented spherical neural mass may be dissociated into single cells by using, as a cell separating enzyme, an enzyme well known in the art, such as trypsin-EDTA, disphase, Accutase, and collagenase. According to an example of the present invention, the dissociation into single cells was performed using Accutase.

According to an example of the present invention, the fragmented spherical neural mass could be dissociated into single cells by attachment and culture on a culture dish with a diameter of 60 mm coated with CellStart using a culture medium supplemented with bFGF and an N2 additive for 12-24 h, removal of the medium, addition of 3-5 ml of Accutase, and then culture in an incubator at 37°C for 1-3 h.

The cystic structure of spherical neural mass may mean a blister-like transparent cyst present in the spherical neural mass, and this may protrude as a blister-like transparent cyst from the spherical neural mass or may be present in a form entirely surrounded in a transparent cyst. The cystic structure of spherical neural mass may be dissociated using a metal wire, a metal net having micrometer (µm)-sized holes, and the like. According to an example of the present invention, the cystic structure of spherical neural mass was dissociated using a tungsten wire.

The cystic structure of spherical neural mass may be fragmented.

### Step of culturing spherical neural mass single cells and cystic structure of spherical neural mass together in single medium

In the present step, spherical neural mass single cells and a cystic structure of spherical neural mass are cultured together in single medium.

In an embodiment of the present invention, in the culturing, the spherical neural mass single cells are disposed in the upper portion and the cystic structure of spherical neural mass is disposed in the lower portion in spaces spaced above and below by a porous structure.

In an embodiment of the present invention, the porous structure is a porous mesh.

In an embodiment of the present invention, the spherical neural mass single cells are separated from a non-cystic structure of spherical neural mass.

In an embodiment of the present invention, the cystic structure of spherical neural mass is separated from a spherical neural mass-derived transparent cyst.

The cystic structure of spherical neural mass may be fragmented and cultured. The cystic structure of spherical neural mass is not limited in its fragmentation size.

The cystic structure of spherical neural mass may be placed on a culture dish coated with CellStart at 15 to 20 cystic structure fragments per culture dish and attached and cultured in DMEM/F12 culture medium supplemented with N2 and B27 additives, but is not limited thereto. The cystic structure of spherical neural mass is disposed in the lower portion in the spaces spaced above and below by a porous structure.

The spherical neural mass single cells may be disposed at 0.5×10⁵ to 1.5×10⁵ on a culture dish coated with CellStart, and then cultured together with the cystic structure of spherical neural mass for 1-3 weeks, but is not limited thereto. The spherical neural mass single cells are disposed in the upper portion in the spaces spaced above and below by the porous structure.

The coating material used in each step may be CellStart, but is not limited thereto, and the extracellular matrix (ECM) for cell attachment may be further contained. The reason is that undifferentiated stem cells and nerve cells cannot be attached and grown themselves onto a culture dish and thus the maintenance culture thereof is possible only with the help of the extracellular matrix or other supporting cells.

In addition to CellStart, the extracellular matrix may be used alone or in combination with other substances.

In an embodiment of the present invention, the spherical neural mass is differentiated from stem cells.

In an embodiment of the present invention, the stem cells are pluripotent stem cells.

In an embodiment of the present invention, the stem cells are selected from the group consisting of embryonic stem cells, induced pluripotent stem cells (iPSCs), adult stem cells, somatic cell nuclear transfer embryonic stem cells, and stem cells generated by direct reprogramming. According to an example of the present invention, the stem cells are embryonic stem cells or induced pluripotent stem cells.

In an embodiment of the present invention, the spherical neural mass is differentiated from stem cells through the following steps:
forming embryoid bodies from the stem cells;
forming neural rosettes and neural tube-like structures from the embryoid bodies; and
forming the spherical neural mass from the neural rosettes and the neural tube-like structures.

The step of forming embryoid bodies from the stem cells may be performed for 4-6 days, but is not limited thereto.

The step of forming embryoid bodies from the stem cells may be performed using Essential 6 cell culture medium, but is not limited thereto.

As used herein, the term "embryoid body" refers to a three-dimensional aggregate of pluripotent stem cells represented by embryonic stem cells, and the pluripotent stem cells can undergo differentiation through the embryoid bodies in the initial embryonic development stage and can differentiate into all the three germ lineages of endoderm, mesoderm, and ectoderm.

The step of forming neural rosettes and neural tube-like structures from the embryoid bodies may be performed for 4-6 days, but is not limited thereto.

The step of forming neural rosettes and neural tube-like structures from the embryoid bodies may be performed using a culture dish coated with CellStart, but is not limited thereto.

The step of forming neural rosettes and neural tube-like structures from the embryoid bodies may be performed by culturing the embryoid bodies, formed in the embryoid body formation process, in a culture dish coated with CellStart using DMEM/F12 minimal culture medium supplemented with only 1/2 N2 additive for 4-6 days, replacing 1/2 N2 supplemented DMEM/F12 minimal culture medium with bFGF and N2 supplemented DMEM/F12 culture medium, and then proliferating through culturing for 5-10 days, but is not limited thereto.

The culture medium used in the present step may be DMEM/F12 cell culture medium supplemented with bFGF and N2 additive, but any culture solution that can form/maintain neural rosettes may be selectively used without limitation.

### Step of differentiating spherical neural mass into retinal outer layer cells

In the present step, the spherical neural mass single cells and the cystic structure of spherical neural mass are differentiated into retinal outer layer cells after the spherical neural mass single cells and the cystic structure of spherical neural mass are cultured together in a single medium.

In an embodiment of the present invention, the spherical neural mass is spherical neural mass single cells, a cystic structure of spherical neural mass, or spherical neural mass single cells and a cystic structure of spherical neural mass.

In an embodiment of the present invention, the retinal outer layer cells are photoreceptor cells, retinal pigment epithelial cells, or photoreceptor cells and retinal pigment epithelial cells.

In an embodiment of the present invention, the retinal outer layer cells have increased expression of rhodopsin and βIII-tubulin or increased expression of RPE65 and bestrophin.

In an embodiment of the present invention, the retinal outer layer cells alleviate or prevent blindness.

In accordance with another aspect of the present invention, there is provided a pharmaceutical composition for prevention or treatment of a retinal disease, the pharmaceutical composition including the retinal outer layer cells generated by the differentiation method.

In an embodiment of the present invention, the retinal disease is selected from the group consisting of retinal dysplasia, retinal degeneration, aged macular degeneration, diabetic retinopathy, retinitis pigmentosa, glaucoma, optic neuropathy, trauma, retinal detachment, Leber congenital amaurosis, and congenital retinal dystrophy.

Since the retinal outer layer cells are the same as the retinal outer layer cells described with respect to the differentiation method, the description of the retinal outer layer cells is omitted to avoid excessive complexity of the specification.

In accordance with another aspect of the present invention, there is provided a method for treating a retinal disease, the method including administering, to a subject, the retinal outer layer cells generated by the differentiation method.

The "subject" refers to an individual in need of treatment of a disease, and more specifically, refers to a mammal such as a human or non-human primate, a mouse, a dog, a cat, a horse, or cow.

In accordance with still another aspect of the present invention, there is provided use of the retinal outer layer cells, generated by the differentiation method, for treating a retinal disease.

Since the retinal outer layer cells are the same as the retinal outer layer cells described with respect to the above-mentioned differentiation method, the description of the retinal outer layer cells is omitted to avoid excessive complexity of the specification.

### Advantageous Effects of Invention

Features and advantages of the present disclosure are summarized as follows.

The present invention provides a method for differentiating a spherical neural mass (SNM) into retinal outer layer cells and a composition containing retinal outer layer cells generated by the differentiation method.

The use of the differentiation method of the present invention can efficiently differentiate neural progenitor cells into retinal outer layer cells by using only the spherical neural mass without other chemical constituents or supporting cells besides culture media and basic culture additives, and thus the retinal outer layer cells can be advantageously used in related research and development and commercialization.

### Brief Description of Drawings

FIG. 1A shows cell morphology at each stage of the differentiation induction of retinal outer layer cells and a culture dish used in isolation culturing according to an example of the present invention.
FIG. 1B shows cell morphology at each stage of the differentiation induction of retinal outer layer cells and a culture dish used in isolation culturing according to an example of the present invention.
FIG. 2 shows the results of analyzing SNM characteristics according to an example of the present invention.
FIG. 3A shows the result of identifying an optimum isolation culturing in the differentiation of retinal outer layer cells (culturing only spherical neural mass single cells).
FIG. 3B shows the result of identifying an optimum isolation culturing in the differentiation of retinal outer layer cells (mix-culturing spherical neural mass simple cells and cystic fragments).
FIG. 3C shows the results of identifying an optimum isolation culturing method for the differentiation of retinal outer layer cells (culturing cystic fragments in a cell culture insert and spherical neural mass single cells in a 6-well cell culture dish for 1 day, followed by isolation culturing).
FIG. 3D shows the results of identifying an optimum isolation culturing method for the differentiation of retinal outer layer cells (culturing spherical neural mass single cells in a cell culture insert and cystic fragments in a 6-well cell culture dish for 1 day, followed by isolation culturing).
FIG. 3E shows the results of identifying an optimum isolation culturing method for the differentiation of retinal outer layer cells (culturing spherical neural mass single cells on the back side of the bottom of a cell culture insert and cystic fragments in a 6-well cell culture dish for 1 day, followed by isolation culturing).
FIG. 4A shows the results of identifying the differentiation or non-differentiation of photoreceptor cells differentiated from embryonic stem cells by the expression or non-expression of markers, according to an example of the present invention.
FIG. 4B shows the results of identifying the differentiation or non-differentiation of photoreceptor cells differentiated from induced pluripotent cells by the expression or non-expression of markers, according to an example of the present invention.
FIG. 4C shows the results of identifying the differentiation or non-differentiation of photoreceptor cells by the measurement of cyclic GMP concentrations.
FIG. 4D shows the results of identifying the differentiation or non-differentiation of photoreceptor cells differentiated from embryonic stem cells by the expression of rhodopsin, βIII-tubulin, and Nestin genes, according to an example of the present invention.
FIG. 4E shows the results of identifying the differentiation or non-differentiation of retinal endothelial cells differentiated from embryonic stem cells by RPE65, Bestrophin, and ZO-1 markers, according to an example of the present invention.
FIG. 4F shows the results of identifying the differentiation or non-differentiation of retinal endothelial cells differentiated from induced pluripotent stem cells by RPE65, Bestrophin, and ZO-1 markers, according to an example of the present invention.
FIG. 5A shows the results of identifying the in vivo engraftment of differentiated photoreceptor cells and retinal endothelial cells through an animal experiment, according to an example of the present invention.
FIG. 5B shows electroretinography performed to measure the efficacy of differentiated photoreceptor cells and retinal endothelial cells, according to an example of the present invention.
FIG. 5C shows the results of identifying the efficacy of differentiated photoreceptor cells and retinal endothelial cells through electroretinography, according to an example of the present invention.

### Best Mode for Carrying out the Invention

Hereinafter, the present invention will be described in more detail with reference to exemplary embodiments. These exemplary embodiments are provided only for the purpose of illustrating the present invention in more detail, and therefore, according to the purpose of the present invention, it would be apparent to a person skilled in the art that these exemplary embodiments are not construed to limit the scope of the present invention.

### EXAMPLES

Throughout the present specification, the "%" used to express the concentration of a specific material, unless otherwise particularly stated, refers to (wt/wt)% for solid/solid, (wt/vol)% for solid/liquid, and (vol/vol)% for liquid/liquid.

### Culturing of human embryonic stem cells (hESCs)

Undifferentiated hESCs (SNUhES32) for differentiation into retinal outer layer cells were cultured in Vitronectin (GIBCO, A27940) coated culture dishes containing an Essential 8 (GIBCO, A15169-01) culture solution.

The undifferentiated stem cells were cultured in a colony form. Under the principle of 7-day culturing, when the center portion of the colony was about to be induced to differentiate, the colony was broken into 20-30 grid fragments by using a tool having a hook shape made from the end of a glass pipette, and 100 fragments were transferred onto new culture dishes coated with Vitronectin and subcultured, and the culture solution was exchanged daily within 24 h every day from the third day to the seventh day.

### Culturing of induced pluripotent stem cells (iPSCs)

Undifferentiated iPSCs (hFSiPS1) for differentiation into photoreceptor cells were cultured by the same method as the hESC culturing method.

### Immunocytochemistry Assay

The cells were fixed in a 4% paraformaldehyde solution for 10 min.

For smooth penetration into the cytoplasm, each antibody was incubated with 0.1% Trition X-100 (in PBS) for 15 min and then incubated with 2% bovine serum albumin (BSA, in PBS) for 1 h at room temperature.

Then, primary antibodies were allowed to bind to the cells at 4°C. Secondary antibodies suitable for the respective primary antibody species were used to identify the cells to which the primary antibodies were bound (see Table 1, below).

Finally, in order to identify cell nuclei, the cells were incubated in PBS containing 4',6-diamino-2-phenylindole (DAPI) for 10 min to complete nucleus staining, which was then imaged under a fluorescence microscope, and important markers were identified and analyzed.

**TABLE 1**

| Protein | Species | Company | Dilution |
|---|---|---|---|
| Nestin | Rabbit | Millipore | 1:100 |
| Musashi | Mouse | Chemicon | 1:200 |
| Pax6 | Rabbit | Novus viologicals | 1:100 |
| | Mouse | Chemicon | 1:200 |
| βIII-tub | Rabbit | BioLegend | 1:1000 |
| | Mouse | BioLegend | 1:500 |
| SOX1 | Rabbit | Chemicon | 1:200 |
| SOX2 | Rabbit | Chemicon | 1:100 |
| LMX1A | Goat | Santa Cruz | 1:100 |
| Nurr1 | Rabbit | Chemicon | 1:200 |
| Rhodopsin | Rabbit | Millipore | 1:100 |
| Blueopsin | Rabbit | Chemicon | 1:200 |
| Redopsin | Rabbit | Chemicon | 1:100 |
| PDE6b | Rabbit | abeam | 1:50 |
| Recoverin | Rabbit | Chemicon | 1:100 |
| ZO-1 | Rabbit | Zymed | 1:200 |
| RPE65 | Mouse | Novus biological | 1:200 |
| Bestrophin | Mouse | Novus biological | 1:200 |

### Differentiation of human pluripotent stem cells into retinal outer layer cells

Undifferentiated human embryonic stem cells (hESCs) or induced pluripotent stem cells (iPSCs), which are pluripotent stem cell lines, were stabilized by two passages of subculture from the time of thawing, and after the third passage of subculture, the culture was maintained for 7 days.

The culture of human embryonic stem cells (hESCs) or induced pluripotent stem cells (iPSCs) (pluripotent stem cells) cultured for 7 days was removed, and the cells were washed once with DPBS (-), subjected to the addition of 1 ml of disphase, and then incubated for 3 min in a 37°C incubator, so that the cells were dissociated. For embryoid body (EB) formation, the separated cells were cultured in an EB medium (Essential 6, 0.5% penicillin, streptomycin) on Petri culture dishes for 5 days.

On the 5th day of EBs, for the growing of only neurogenic cells, EBs were transferred to CellStart-coated dishes and cultured for 5 days in MEM/F12 medium (containing 0.5% N2 substrate, 2 mM L-glutamine, 1% nonessential amino acids (NEAA), 0.5% penicillinstreptomycin, and 0.1 mM beta-mercaptoethanol), which was a neural selection medium. Furthermore, for the formation and proliferation of neural rosettes and neural tube-like structures, the EBs were cultured for 4-7 days in DMEM/F12 medium (containing 0.5% N2 substrate, 2 mM L-glutamine, 20 ng/ml basic fibroblast growth factor (bFGF), 1% nonessential amino acids (NEAA), 0.5% penicillinstreptomycin, and 0.1 mM beta-mercaptoethanol), which was a proliferation medium.

Thereafter, for the formation and proliferation of neural rosettes and neural tube-like structures, the EBs were cultured for 10-14 days in N2bF medium (containing DMEM/F12 medium, 1% N2 substrate, 2 mM L-glutamine, 20 ng/ml basic fibroblast growth factor (bFGF), 1% nonessential amino acids (NEAA), 0.5% penicillinstreptomycin, and 0.1 mM beta-mercaptoethanol).

When neural rosettes and neural tube-like structures were observed during culturing, the neural rosettes and neural tube-like structures were detached and cut from the bottom by using a fire-polished glass Pasteur pipette, and thereafter, transferred onto petri dishes and cultured in N2bF medium to form a spherical neural mass (SNM).

The subculture of SNM was carried out by mechanically cutting with a 0.1 mm tungsten wire when the diameter of SNM reached about 500 µm, and passaging was possible up to 10 times without changing characteristics.

One day before isolation culturing for differentiation into retinal outer layer cells, a spherical neural mass-derived cystic structure was attached and cultured on CellStart-coated 6-well culture dishes, and single cells dissociated from a non-cystic structure of spherical neural mass (spherical neural mass single cells) were attached and cultured on CellStart-coated cell culture inserts. The dissociation into SNM single cells was carried out by attaching and culturing fragmented SNM on CellStart-coated culture dishes and, after one day, performing Accutase treatment. N2B27 cell culture medium was used for cell culturing for isolation culturing.

The isolation culturing was carried out by placing cell culture inserts, in which spherical neural mass single cells were cultured, into 6-well culture dishes in which cystic structure fragments were cultured, and N2B27 cell culture medium was used for cell culturing, and the medium was replaced every two days for 3 weeks of isolation culturing until the differentiation was ended.

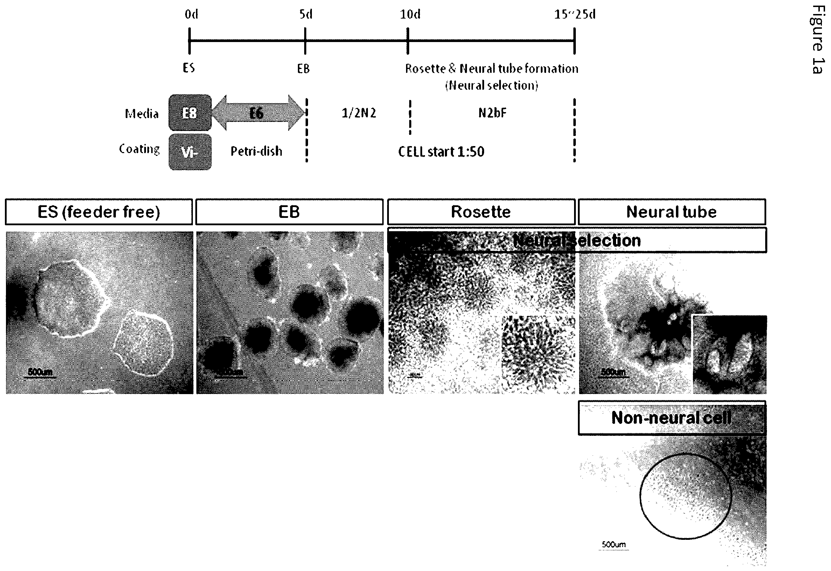

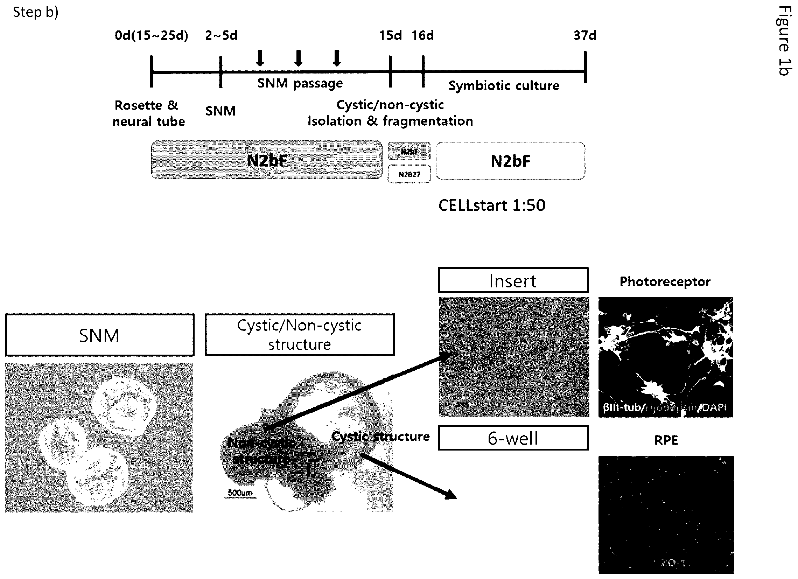

FIGS. 1A and 1B are simple diagram of the above procedures according to days and cell types according to stages.

### Optimization of retinal outer layer cell differentiation

In order to find out culture conditions optimized for the differentiation into retinal outer layer cells from cells completing the differentiation into SNM, culturing was performed under each of the following conditions.
a) culturing only SNM cells
b) mix-culturing SNM cells and cystic fragments
c) culturing cystic fragments in a cell culture insert and SNM single cells in a 6-well cell culture dish for 1 day, followed by isolation culturing
d) culturing SNM single cells in a cell culture insert and cystic fragments in a 6-well cell culture dish for 1 day, followed by isolation culturing
e) culturing SNM single cells on the back side of the bottom of a cell culture insert and cystic fragments in a 6-well cell culture dish for 1 day, followed by isolation culturing

Culturing under the above conditions was performed on CellStart-coated culture dishes, and N2B27 medium was used for the cell culture medium.

### Reverse transcription polymerase chain reaction (RT-PCR)

To identify the differentiation or non-differentiation into retinal outer layer cells according to the period of time of separate culture, RT-PCR analysis for the expression, at the transcriptional level, of rhodopsin, a marker expressed in photoreceptor cells among the retinal outer layer cells was performed as follows.

RNA of photoreceptor cells differentiated from SNM was obtained using TRIzol Reagent (Invitrogen, Carlsbad, CA, USA), and cDNA synthesis was performed using oligo-dT as a primer according to the manufacturer's instructions (AccuPower RT PreMix; Bioneer, Taejeon, Korea) using AMV reverse transcriptase (RT).

PCR amplification was performed using Taq Polymerase (HiPi Plus 5x PCR Premix; Elpis biotech, Taejeon, Korea), and a standard method was used. PCR conditions were: denaturation at 94°C for 5 min, and 30 cycles of 94°C for 30 s, 55°C for 30 s, and 72°C for 10 s. To analyze relative expression with other mRNAs, the amount of cDNA was normalized based on the signal of glyceraldehyde-3-phosphate dehydrogenase (GAPDH) mRNA.

As a result of repeated experiments under various conditions of annealing temperatures and number of cycles, proportionately amplified sections were determined for each primer pair, and PCR conditions were optimized.

The primer sequences used for gene expression analysis are shown in Table 2 below.

**TABLE 2**

| Gene name | Gene direction | Nucleotide sequence | Sequence number |
|---|---|---|---|
| Rhodopsin | Forward | 5'-CACAGGATGCAATTTGGAGG- 3' | SEQ ID NO: 1 |
| | Reverse | 5'-CCTTCTGTGTGGTGGCTGAC- 3' | SEQ ID NO: 2 |
| βIII-tubulin | Forward | 5'-CAACAGCACGGCCATCCAGG- 3' | SEQ ID NO: 3 |
| | Reverse | 5'-CTTGGGGCCCTGGGCCTCCGA- 3' | SEQ ID NO: 4 |
| Nestin | Forward | 5'-CAGCTGGCGCACCTCAAGATG- 3' | SEQ ID NO: 5 |
| | Reverse | 5'- AGGGAAGTTGGGCTCAGGACTGG- 3' | SEQ ID NO: 6 |
| GAPDH | Forward | 5'-ACCACAGTCCATGCCATCAC- 3' | SEQ ID NO: 7 |
| | Reverse | 5'-TCCACCACCCTGTTGCTGTA- 3' | SEQ ID NO: 8 |

### Measurement of concentration of photoconduction pathway metabolites

The cGMP Enzyme immunoassay kit (GE Healthcare) was used according to the manufacturer's protocol to measure the concentration of photoconduction pathway metabolites in the retinal outer layer cells on the 7th day of separate culture. The cells were treated with a phosphodiesterase inhibitor (IBMX, 50 mM) 48 h before the cGMP measurement.

### Transplantation of SNM-derived retinal outer layer cells in retinal degeneration rodent model

Six-week-old SD rats were intravenously injected with a sterilized 1% NaIO₃ saline solution (70 mg/kg). Four days after the intravenous injection, the animals were anesthetized by the intramuscular injection of tiletamine/zolazepam (Zoletil^{®}) and xylazine.

The pupils were dilated by 0.5% tropicamide and phenylephrine HCl 2.5% eye drops (Mydrin-P^{®}) and treated with a proparacaine HCl 0.5% (Alcaine^{®}) topical ophthalmic anesthetic.

Prior to the transplantation, the SNM-derived retinal outer layer cells were incubated with DAPI (10 µg/ml) for 30 min. Several times of washing were performed to remove DAPI from the media, and the cells were separated by culture under 0.05% trypsin/0.1% EDTA for 5 min at 37°C.

The separated retinal outer layer cells (1×10⁵ cells/2 ul) visualized through a surgical microscope was injected into the subretinal space or the vitreous cavity by using a 26-gauge needle attached to a Hamilton syringe.

Cyclosporine A (210 mg/l, Cipol-N^{®}, Chong Kun Dang, Seoul, Korea) was provided in drinking water one day before transplantation until the enucleation procedure. One week after transplantation, the eyes were enucleated and quickly frozen by using an embedding compound (FSC 22^{®}, Leica microsystems, IL).

The cooled pieces were washed with PBS and blocked in a 3% bovine serum albumin solution in PBS containing 0.1% Triton X100. After blocking, the pieces were incubated with primary antibodies for one day. For immunohistochemical examination, anti-bIII-tub antibody and mixed human antibody, RPE65 and ZO-1 antibodies were used.

For electroretinography, b-wave was measured on 4, 12, and 24 weeks after cell transplantation.

### Results

### Differentiation of retinal outer layer cells from human pluripotent stem cells

FIGS. 1A and 1B show schematic diagrams of a series of procedures of the present invention and cell morphology at each stage.

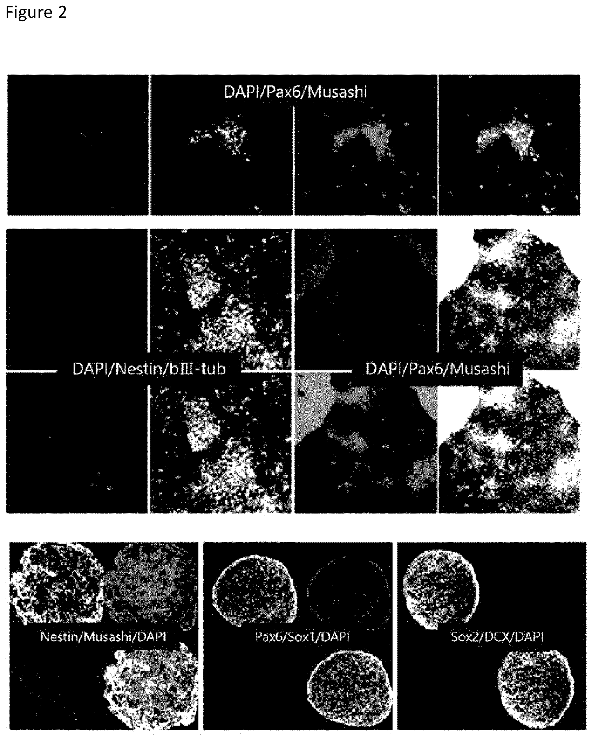

In order to differentiate pluripotent stem cells (including embryonic stem cells and induced pluripotent stem cells) into retinal outer layer cells, the differentiation of pluripotent stem cells into nervous system cells needs to be induced. From the beginning, the culture structures and cell culture media were used to induce efficient differentiation into neural progenitor cells via selective culture of neural progenitor cells, growth culture, induction of nerve-specific structures, and formation of a spherical neural mass. It could be seen that, unless a separate differentiation signal was given, the spherical neural mass maintained the properties of neural progenitor cells at the initial stage and even after repeated passages (FIG. 2).

It was finally confirmed that only two types of structures of spherical neural mass were isolation cultured to enable efficient differentiation into photoreceptor cells and retinal epithelial cells, which were the targets of the present invention.

It was confirmed in the present invention that in the differentiation into photoreceptor cells and retinal epithelial cells using pluripotent stem cells, the efficient of differentiation can be dramatically increased by even only a culture method, without medium supplements, such as external cellular transduction signal activators and inhibitors.

### Optimization of retinal outer layer cell differentiation

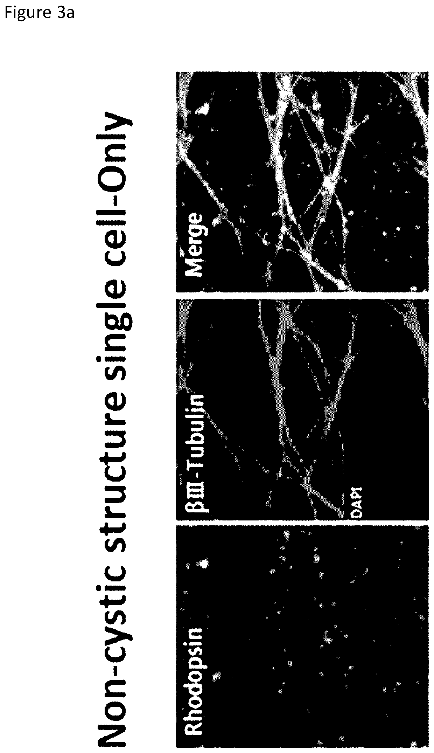

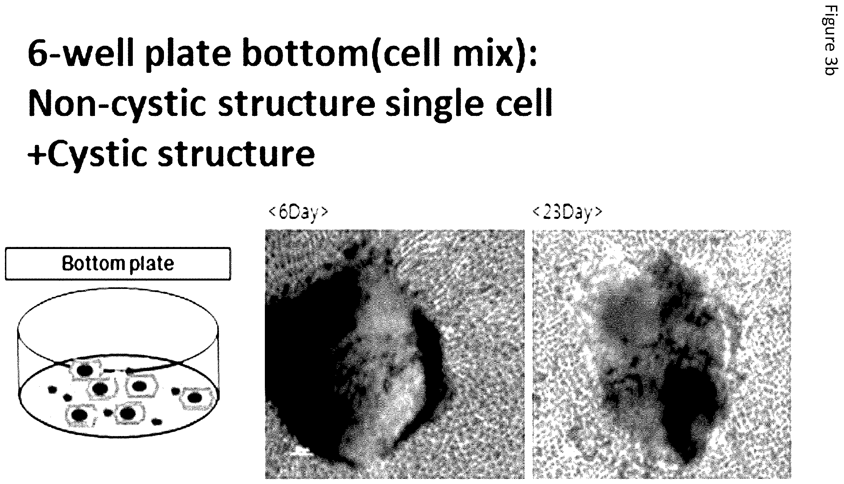

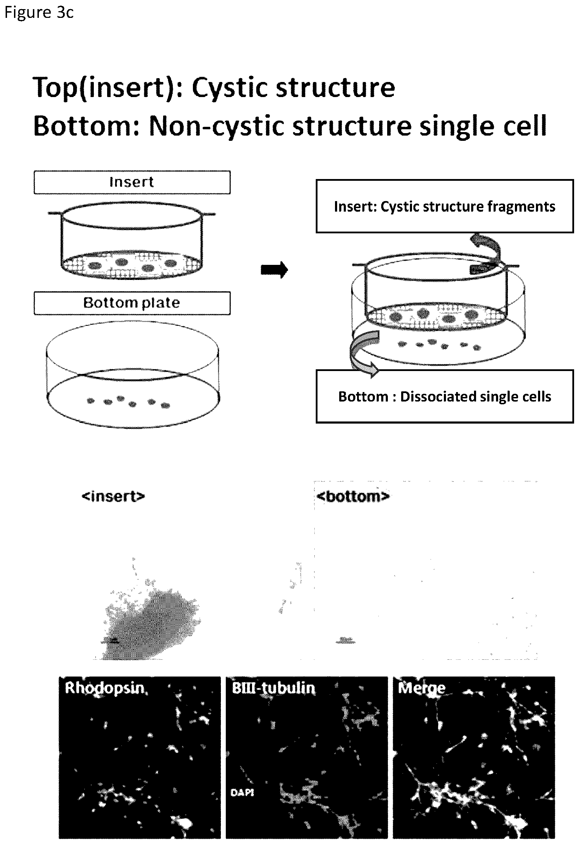

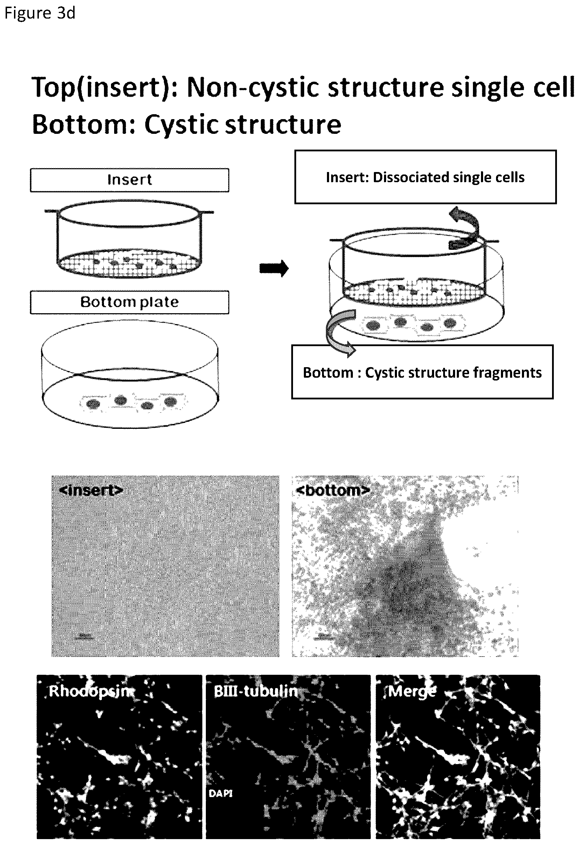

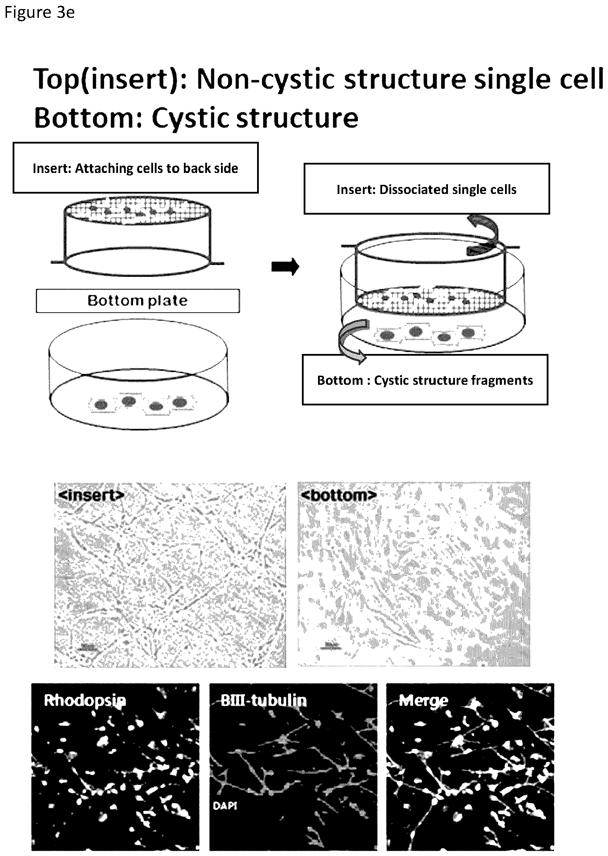

Considering the section "optimization of retinal outer layer cell differentiation" above, in differentiation condition a), the expression of rhodopsin, a marker of photoreceptor cells, was hardly observed (FIG. 3A), and in differentiation condition b), only cystic structure-derived cells survived and the spherical neural mass single cells almost hardly survived (FIG. 3B). In culture condition c), the expression of rhodopsin was observed in the spherical neural mass single cells (FIG. 3D) but showed a low tendency compared with culture condition d). In culture condition d), the expression of rhodopsin was highest (FIG. 3D). In culture condition e), the expression of rhodopsin in spherical neural mass single cells showed a similar tendency compared with that in culture condition d) (FIG. 3E), but the spherical neural mass single cells were attached to the back side of the cell culture insert, and thus the mixing with a cystic structure could not be completely excluded, and the recovery of each was not easy, and thus culture condition d) was optimal for the differentiation into retinal outer layer cells (photoreceptor cells).

### Functional characterization of retinal outer layer cells derived from pluripotent stem cells

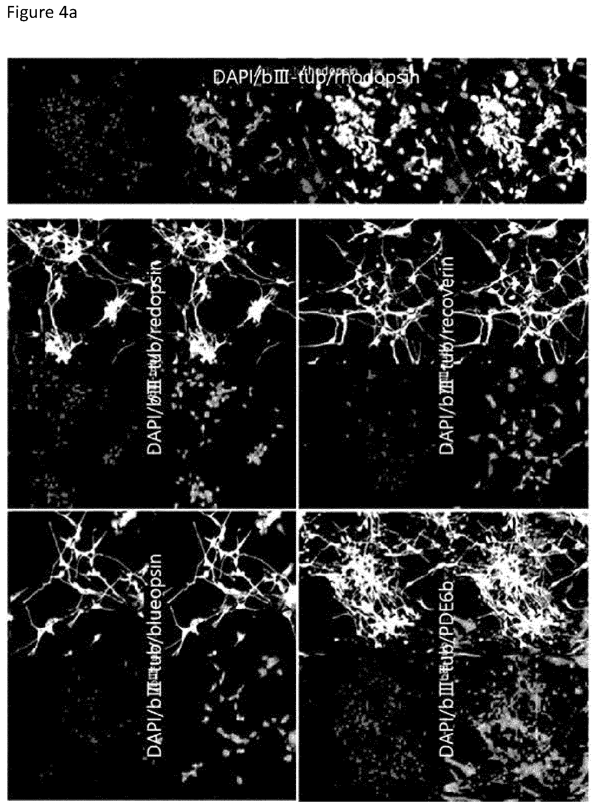

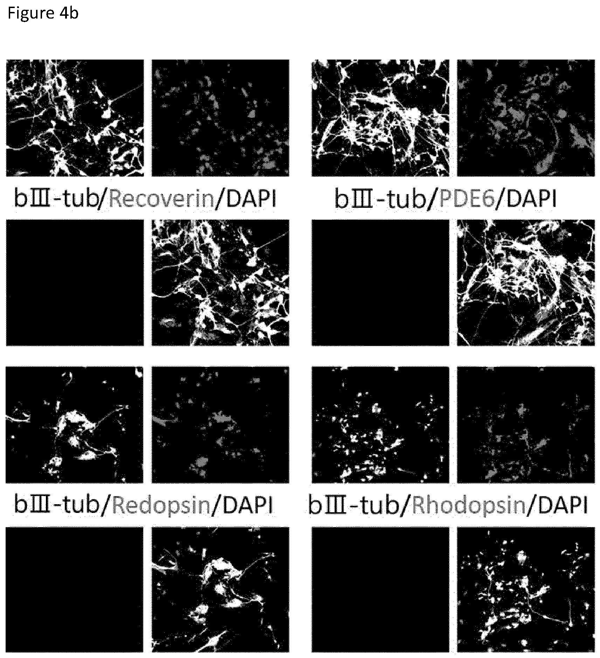

Specific molecular markers related to cellular functions of mature photoreceptor cells were identified using immunocytochemistry. As can be seen in FIGS. 4A and 4B, the cells that have been differentiated from embryonic stem cells or induced pluripotent stem cells expressed blueopsin, redopsin, PDE6b, recoverin, and rhodopsin, and also expressed βIII-tubulin, a marker for neurons.

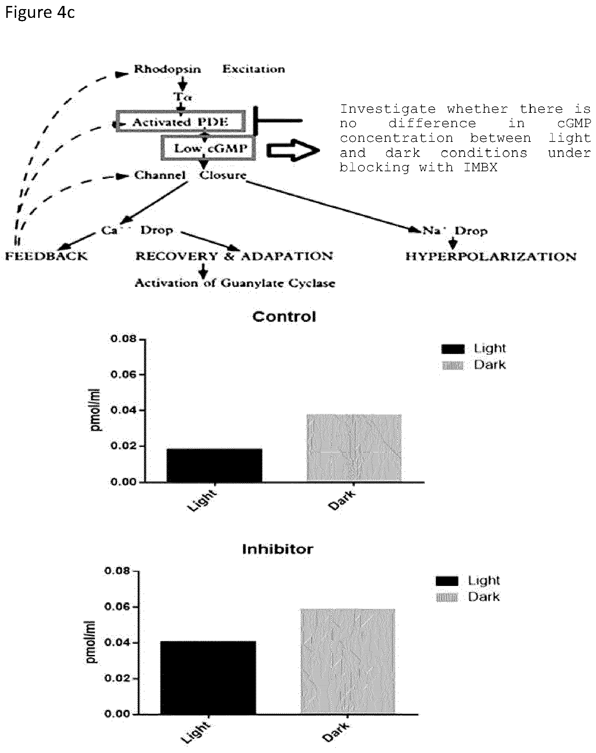

The *in vitro* evaluation of functions of photoreceptor cells may be performed by determining a difference in the concentration of a photoconduction pathway metabolite measured under the light and dark conditions. There is a difference in cyclic GMP concentration in the functioning photoconduction pathway, and when the differentiated photoreceptor cells were cultured in the light and the dark for the same period, the concentration of cyclic GMP was lower in the cells cultured in the light than in the cells cultured in the dark. The same pattern was also observed when the cells were treated with a phosphodiesterase inhibitor (IBMX). This shows that the photoreceptor cells differentiated in the present invention contained a photoconduction pathway, meaning that the differentiation into photoreceptor cells was attained (FIG. 4C).

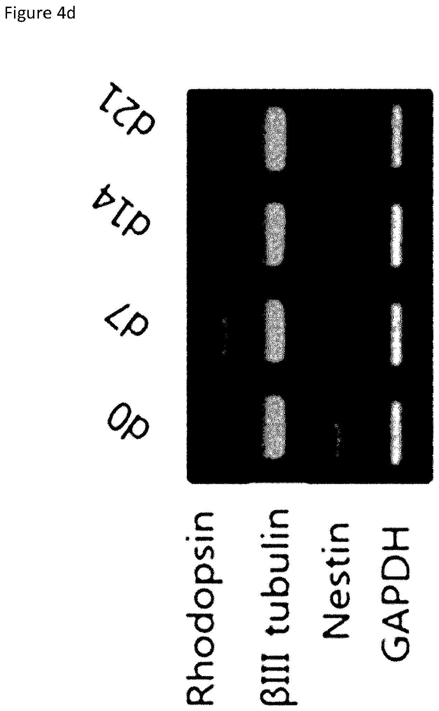

The expression of rhodopsin, a specific protein of photoreceptor cells, during the isolation culture was identified by RT-PCR, and as a result, the mRNA expression of rhodopsin was highest on the seventh day of isolation culture. These results confirmed that the differentiation into photoreceptor cells already occurred at a high level on day 7 of isolation culture, and Nestin, a marker of neural progenitor cells, was hardly expressed on day 21, indicating that most of the cells had been differentiated (FIG. 4D).

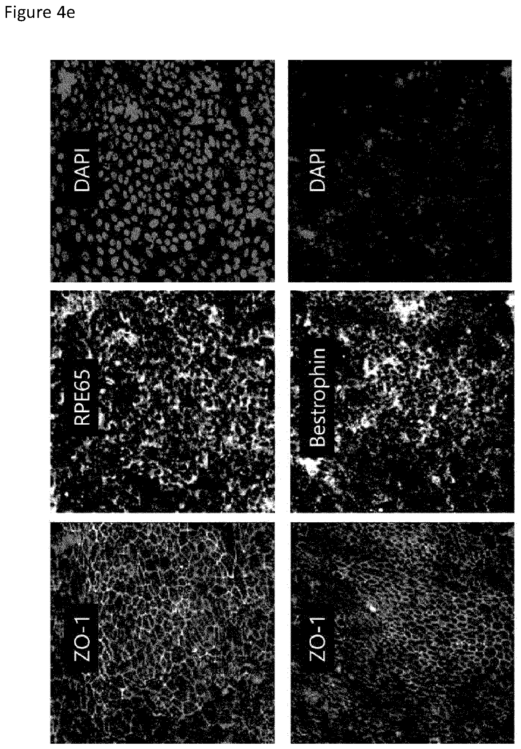

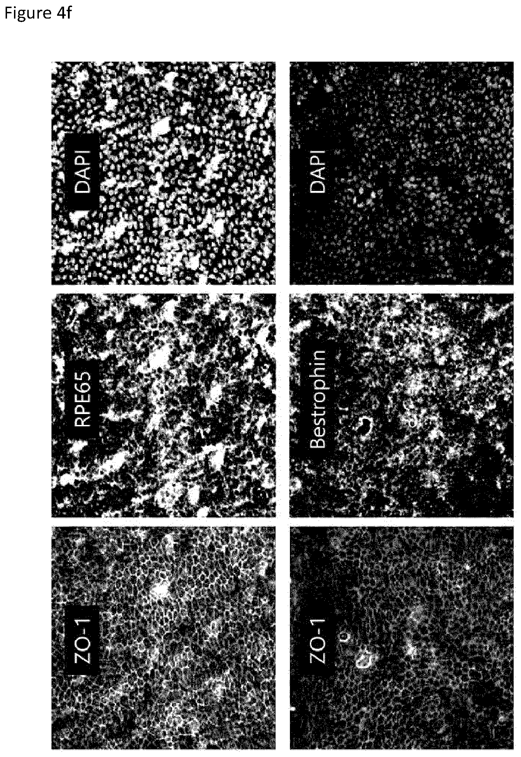

The differentiation or non-differentiation into retinal pigment epithelial cells was investigated using immunocytochemistry using specific molecular markers. As can be confirmed in FIGS. 4E and 4F, the retinal pigment epithelial cells that had been differentiated from embryonic stem cells or induced pluripotent stem cells expressed ZO-1, RPE65, and Bestrophin.

### Effects of transplantation of SNM-derived retinal outer layer cells in retinal degeneration rodent model

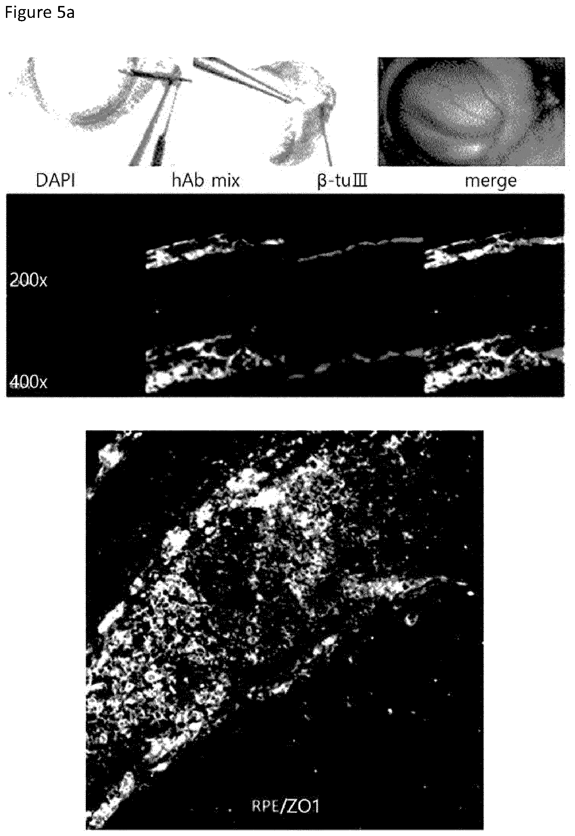

Retinal degeneration rats were transplanted with photoreceptor cells and retinal pigment epithelial cells differentiated based on the present invention and, after 12 weeks, investigated for engraftment. As a result, it was confirmed that human antibody and βIII-tubulin, a marker of neurons, were expressed together with DAPI. RPE65 and Bestrophin, markers of retinal endothelial cells, were expressed together with ZO-1, a marker of endothelial cells (FIG. 5A). Furthermore, all the transplanted cells were observed in the retinal outer layer, indicating that the transplanted cells had high engraftment.

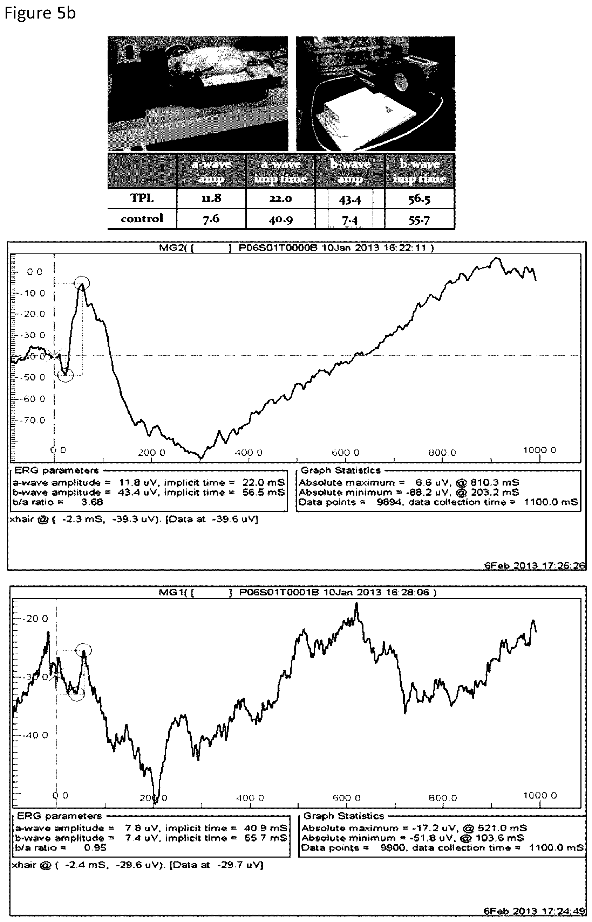

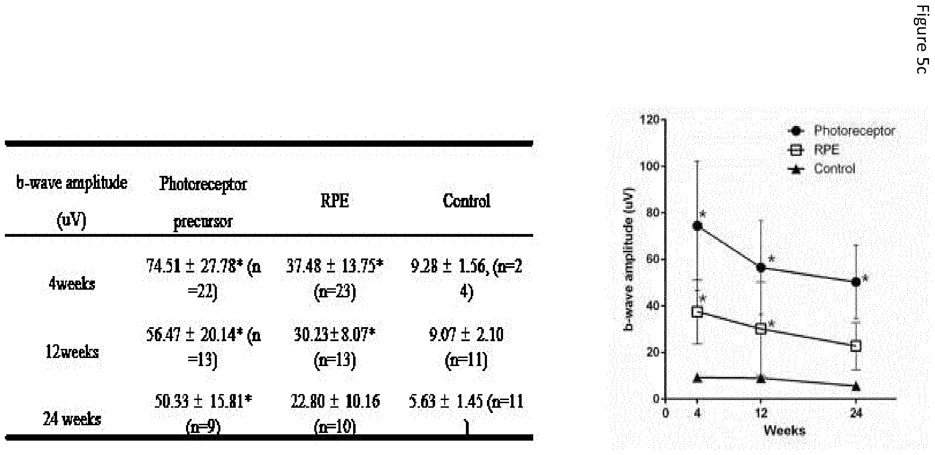

The electroretinography results confirmed that b-wave amplitudes were maintained in all the transplantation groups (FIG. 5B) and as time passed after transplantation, the visual function was slightly reduced but significantly increased compared with a control group. Additionally, the transplantation of photoreceptor cells showed a higher visual function improvement compared with RPE transplantation (FIG. 5C).

### Industrial Applicability

The present invention relates to a method for differentiation and production of retinal outer layer cells from a spherical neural mass (SNM).

## Claims

1. A method for differentiating a spherical neural mass (SNM) into retinal outer layer cells, the method comprising:
culturing spherical neural mass single cells and a cystic structure of spherical neural mass together in a single medium; and
differentiating the spherical neural mass into retinal outer layer cells.

2. The method of claim 1, wherein in the culturing, the spherical neural mass single cells are disposed in the upper portion and the cystic structure of spherical neural mass is disposed in the lower portion in spaces spaced above and below by a porous structure.

3. The method of claim 2, wherein the porous structure is a porous mesh.

4. The method of claim 1, wherein the spherical neural mass single cells are isolated from a spherical neural mass-derived noncystic structure.

5. The method of claim 1, wherein the cystic structure of spherical neural mass is isolated from a spherical neural mass-derived transparent cyst.

6. The method of claim 1, wherein the spherical neural mass is differentiated from stem cells.

7. The method of claim 6, wherein the stem cells are selected from the group consisting of embryonic stem cells (ESCs), induced pluripotent stem cells (iPSCs), adult stem cells, somatic cell nuclear transfer embryonic stem cells, and stem cells generated by direct reprogramming.

8. The method of claim 1, wherein the spherical neural mass is differentiated from stem cells through the following steps:
forming embryoid bodies from the stem cells;
forming neural rosettes and neural tube-like structures from the embryoid bodies; and
forming the spherical neural mass from the neural rosettes and the neural tube-like structures.

9. The method of claim 1, wherein the retinal outer layer cells have increased expression of rhodopsin and βIII-tubulin or increased expression of RPE65 and bestrophin.

10. The method of claim 1, wherein the retinal outer layer cells are photoreceptor cells, retinal pigment epithelial cells, or photoreceptor cells and retinal pigment epithelial cells.

11. The method of claim 1, wherein the retinal outer layer cells alleviate or prevent blindness.

12. A pharmaceutical composition for prevention or treatment of a retinal disease, the pharmaceutical composition comprising the retinal outer layer cells generated by the differentiation method of claim 1.
